# EUROPEAN PATENT SPECIFICATION

(11) **EP 1 723 240 B1**
(45) Date of publication and mention of the grant of the patent: **11.08.2010**
(21) Application number: 04712389.8
(22) Date of filing: 18.02.2004
(51) Int. Cl.: C12N 15/64, C12M 3/00, C12M 1/14

(54) **CULTURE DEVICE AND METHOD FOR EUKARYOTIC CELL TRANSFECTION**
KULTURVORRICHTUNG UND VERFAHREN ZUR TRANSFEKTION EUKARYONTISCHER ZELLEN
DISPOSITIF DE CULTURE ET PROCEDE DE TRANSFECTION DE CELLULE EUCARYOTE

(43) Date of publication of application: 22.11.2006
(73) Proprietor: NITTO DENKO CORPORATION, Ibaraki Osaka 567-8680 (JP)
(72) Inventor: TANAKA, Yasunobu, San Diego, California 92122 (US); YU, Lei, Carlsbad, California 92008 (US); JI, Shouping, Oceanside, California 92054 (US)
(74) Representative: Miles, John Stephen
(86) International application number: PCT/US2004/004818
(87) International publication number: WO 2005/083091

(56) References cited:
- EP-A- 1 398 042
- US-A- 5 861 306
- US-B1- 6 544 790
- ZHENG J ET AL: "TRANSFECTION OF CELLS MEDIATED BY BIODEGRADABLE POLYMER MATERIALS WITH SURFACE-BOUND POLYETHYLENEIMINE" BIOTECHNOLOGY PROGRESS, XX, XX, vol. 16, 2000, pages 254-257, XP001145881 ISSN: 8756-7938
- CHANG FU-HSIUNG ET AL: "Surfection: a new platform for transfected cell arrays." NUCLEIC ACIDS RESEARCH 2004, vol. 32, no. 3, 18 February 2004 (2004-02-18), page e33, XP002437066 ISSN: 1362-4962

## Description

### Background of the Invention

### Field of the Invention

The present invention is directed to methods of cell transfection using a transfection device.

### Description of the Related Art

Gene transfection methods can be used to introduce nucleic acids into cells and are useful in studying gene regulation and function. High throughput assays that can be used to screen large sets of DNAs to identify those encoding products with properties of interest are particularly useful. Gene transfection is the delivery and introduction of biologically functional nucleic acids into a cell, such as a eukaryotic cell, in such a way that the nucleic acid retains its function within the cell. Gene transfection is widely applied in studies related to gene regulation, gene function, molecular therapy, signal transduction, drug screening, and gene therapy studies. As the cloning and cataloging of genes from higher organisms continues, researchers seek to discover the function of the genes and to identify gene products with desired properties. This growing collection of gene sequences requires the development of systematic and high-throughput approaches to characterizing gene products and analyzing gene function, as well as other areas of research in cell and molecular biology.

Both viral and non-viral gene carriers have been used in gene delivery. Viral vectors have been shown to have higher transfection efficiency than non-viral carriers, but the safety of viral vectors hampers its applicability (Verma I.M and Somia N. Nature 389 (1997), pp. 239-242; Marhsall E. Science 286 (2000), pp. 2244-2245). Although non-viral transfection systems have not exhibited the efficiency of viral vectors, they have received significant attention, because of their theoretical safety when compared to viral vectors. In addition, viral vector preparation is a complicated and expensive process, which limits the application of viral vectors *in vitro.* The preparation of non-viral carriers is simpler and more cost effective in comparison to preparation of viral carriers, making synthetic gene carriers desirable as transfection reagents in *in vitro* studies.

Most non-viral vectors mimic important features of viral cell entry in order to overcome cellular barriers, which are meant to prevent infiltration by foreign genetic material. Non-viral gene vectors, based on a gene carrier backbone, can be classified as a) lipoplexes, b) polyplexes, and c) lipopolyplexes. Lipoplexes are assemblies of nucleic acids with a lipidic component, which is usually cationic. Gene transfer by lipoplexes is called lipofection. Polyplexes are complexes of nucleic acids with cationic polymer. Lipopolyplexes comprise both a lipid and a polymer component. Often such DNA complexes are further modified to contain a cell targeting or an intracellular targeting moiety and/or a membrane-destabilizing component, for example, a viral protein or peptide or a membrane-disruptive synthetic peptide. Recently, bacteria and phages have also been described as shuttles for the transfer of nucleic acids into cells.

Most non-viral transfection reagents are synthetic cationic molecules and have been reported to "coat" the nucleic acid by interaction of the cationic sites on the cation and anionic sites on the nucleic acid. The positively-charged DNA-cationic molecule complex interacts with the negatively charged cell membrane to facilitate the passage of the DNA through the cell membrane by non-specific endocytosis. (Schofield, Brit. Microencapsulated. Bull, 51(1):56-71 (1995)). In most conventional gene transfection protocols, the cells are seeded on cell culture devices 16 to 24 hours before transfection. The transfection reagent (such as a cationic polymer carrier) and DNA are usually prepared in separate tubes, and each respective solution is diluted in medium (containing no fetal bovine serum or antibiotics). The solutions are then mixed by carefully and slowing adding one solution to the other while continuously vortexing the mixture. The mixture is incubated at room temperature for 15-45 minutes to allow the transfection reagent-DNA complexes to form. Prior to transfection, the cell culture medium is removed and the cells are washed with buffer to remove the residues of serum and antibiotics. The solution containing DNA-transfection reagent complexes is added to the cells, and the cells are incubated for about 3-4 hours. The medium containing transfection reagent would then be replaced with fresh medium. The cells would finally be analyzed at one or more specific time point(s). This is obviously a time consuming procedure, particularly when the number of samples to be transfected is very large.

Several major problems exist in conventional transfection procedures. First, conventional procedures are time-consuming, particularly when there are many cell or gene samples to be used in transfection experiments. Also, the results derived from common transfection procedures are difficult to reproduce, due to the number of steps required. For instance, in producing the DNA-transfection reagent, the formation of the complex is influenced by concentration and volume of nucleic acid and reagents, pH, temperature, type of buffer(s) used, length and speed of vortexing, incubation time, and other factors. Although the same reagents and procedure may be followed, different results may be obtained. Results derived from multi-step procedures are often influenced by human or mechanical error or other variations at each step. In addition, refreshing the cell culture medium following transfection disturbs the cells and may cause them to detach from the surface on which they are cultured, thus leading to variation and unpredictability in the final results. Due to all the factors noted, conventional transfection methods require a highly skilled individual to perform the transfection experiment or assay.

Sabatini (U.S. 2002/0006664A1) describes DNA containing a mixture and deposited on a glass slide. However the system only allows transfection with the previously deposited DNA.

U.S. Patent application no. 10/341,059 describes a cell culture/transfection device where the transfection is mediated by a lipid polymer. Some of the methods of application number 10/341,059 may be applied to the transfection formulations described herein.

A cell culture/transfection device is disclosed herein which reduces the cost of transfection assays. Transfection with a molecule of interest is accomplished in a simple procedure.

As discussed above, conventional transfection is a lengthy and technically difficult procedure. Generally, three steps are required: 1) cells are seeded in cell culture plate or dish and incubated until sufficient confluence is achieved; 2) transfection reagent / nucleic acid complexes are prepared; and 3) nucleic acids of interest are added along with the transfection reagent and further incubation is carried out. Two incubation periods are needed and typically it takes more than two days to complete all the steps. In contrast, embodiments of the present invention provide a simple procedure that involves only a single incubation step. A cell culture device, which has previously been coated with a transfection reagent, allows transfection by adding the nucleic acid of interest and the cell culture in succession. The transfected cells may then be cultured in the same device. Thus the cells may be transfected and cultured in the cell culture device without the need for further manipulation of the cells immediately after the transfection step. Transfection efficiency is comparable to regular transfection. Consequently, this invention provides an easy transfection procedure and reduces the time required for the transfection procedure by more than one day. Large numbers of cells may be transfected efficiently.

### Summary of the Invention

The invention is directed at a method for transfection of eukaryotic cells which includes the steps of providing a solid surface at least partially coated with a calcium metal salt optionally in a gel matrix, adding at least one nucleic acid to be introduced into the eukaryotic cell onto the solid surface, and seeding eukaryotic cells onto the solid surface at a sufficient density and under appropriate conditions for introduction of the nucleic acids into the eukaryotic cells. Preferably, the surface is selected from flasks, dishes, tubes, continuous surface, multi-well plates, slides, and implanted devices. Preferably, the solid surface is glass, polystyrene or epoxy resin.

Preferably, the calcium salt is calcium chloride or calcium acetate.

In preferred embodiments, the calcium metal salt also includes a matrix complex. Preferably, the calcium metal salt is retained on the solid surface. More preferably, the calcium metal salt is retained on the solid surface with a matrix complex. In yet more preferred embodiments, the matrix complex is selected from proteins, glycoproteins, peptides, polysaccharides, and polymers or combinations thereof. Most preferably, the protein is selected from gelatin, collagen, laminin, fibronectin, and bovine serum albumin or a combination thereof. Most preferably, the polymer is selected from hydrogels, biodegradable polymers, and biocompatible materials.

In more preferred embodiments, the solid surface is either a slide or a multi-well plate.

In preferred embodiments, the eukaryotic cells are mammalian cells. In preferred embodiments, the eukaryotic cells are dividing cells or non-dividing cells. In preferred embodiments, the eukaryotic cells are transformed cells or primary cells. In preferred embodiments, the eukaryotic cells are somatic or stem cells. More preferably, the eukaryotic cell is a plant cell. In an alternate more preferred embodiment, the eukaryotic cell is an insect cell.

In preferred embodiments of the method, at least one nucleic acid is added which is selected from DNA, RNA, DNA/RNA hybrid and chemically modified nucleic acids. More preferably, the chemically modified nucleic acid includes a peptide nucleic acid. More preferably, the DNA is circular, linear, or single strand oligonucleotide. More preferably, the RNA is single stranded or double stranded. In a most preferred embodiment, the single-stranded RNA is a ribozyme. In another most preferred embodiment, the double-stranded RNA is siRNA.

The invention may use a multiwell plate for transfecting a eukaryotic cell wherein the bottom of at least some of the wells are at least partially coated with a calcium metal salt. In preferred embodiments, the calcium salt is either calcium chloride or calcium acetate.

In some preferred embodiments, the multiwell plate includes a matrix complex. Preferably, the calcium metal salt is retained on the multiwell plate. More preferably, the calcium metal salt is retained on the multiwell plate with a matrix complex. In more preferred embodiments, the matrix complex is selected from proteins, glycoproteins, peptides, polysaccharides, and polymers or combinations thereof. In some preferred embodiments, the protein is gelatin, collagen, laminin, fibronectin, or bovine serum albumin or a combination thereof. In preferred embodiments, the polymer is selected from hydrogels, biodegradable polymers, and biocompatible materials.

The invention may use a cell culture/transfection device for transfecting a eukaryotic cell, including a solid surface, wherein the solid surface is coated with calcium chloride in a gel matrix. In preferred embodiments, the surface may be a continuous surface, flasks, dishes, tubes, multi-well plates, slides, or implanted devices. In preferred embodiments, the solid surface is glass, polystyrene or epoxy resin. In most preferred embodiments, the solid surface is either a slide or a multi-well plate.

Further aspects, features and advantages of this invention will become apparent from the detailed description of the preferred embodiments which follow.

### Brief Description of the Drawings

These and other feature of this invention will now be described with reference to the drawings of preferred embodiments which are intended to illustrate and not to limit the invention.
Figure 1 shows transfection efficiencies for 293 cells assayed at 0-0.5 mg gelatin/well and 0.25-1.0 µmol CaCl₂/well using GFP as reporter.
Figure 2 shows transfection efficiencies for COS-7 cells assayed at 0-0.5 mg gelatin/well and 0.25-1.0 µmol CaCl₂/well using GFP as reporter.
Figure 3 shows transfection efficiencies for HeLa cells assayed at 0-0.5 mg gelatin/well and 0.25-1.0 µmol CaCl₂/well using GFP as reporter.
Figure 4 shows inhibition of Luciferase activity by siRNA delivery at 0.25 µmol, 0.5 µmol, and 0.75 µmol CaCl₂ concentrations and 36 nM and 0.12 µM concentration of siRNA.
Figure 5 shows efficiencies for co-transfection using GFP plasmid as assayed in three different cell types: 293, COS-7, and HeLa cells.
Figure 6 shows efficiencies for co-transfection using luciferase plasmid as assayed in three different cell types: 293, COS-7, and HeLa cells.
Figure 7 shows epifluorescent micrographs of transfected cells in both small scale (96-well plates and 24-well plates) and large scale (6 well plates and 10 cm dishes) applications. Transmitted light is shown on the left panels and the fluorescence from GFP is shown in the right hand panels.

### Detailed Description of the Preferred Embodiment

A method is described herein which is simple, convenient, and efficient compared to conventional transfection assays. A transfection device is made according to methods described herein by affixing a calcium metal salt on the solid surface of a cell culture device. By using this device, researchers need only add a nucleic acid to the surface of the cell culture device. There is no need to pre-mix the DNA with a transfection reagent. This removes a key timing-consuming step, which is required by conventional transfection procedures. Scientists only require approximately 40 minutes to complete the entire transfection process for 10 samples, compared to 2 to 5 hours or more required by current methods. This is particularly favorable for high throughput transfection assays, in which hundreds of samples will be tested at a time.

As compared to conventional transfection, there are several advantages to the new method described herein. It provides a transfection device that is very easy to store, and it provides a simple method for biomolecule delivery or gene transfection in which no biomaterial/transfection reagent mixing step is required. The transfection procedure described herein can be finished in a short period of time, for instance approximately 40 minutes, and it provides a high throughput method for transfection in which large numbers of samples may be transfected at a time.

Embodiments of the method for gene delivery which are described herein overcome the common problems encountered in conventional transfection assays described above. Calcium metal salt is simply coated onto the surface of a cell culture device, which can be easily commercialized and mass-produced. Customers, researchers for instance, need only add a nucleic acid of interest, directly to the surface of a cell culture device in order to prepare the device prior to transfection. Cells are then seeded on the surface of the cell culture device and incubated, without changing the medium, and the cells are analyzed. Changing medium during the transfection procedure is unnecessary. The methods described herein dramatically reduce the risk of error, by reducing the number of steps involved, thus increasing consistency and accuracy of the system.

According to the methods described herein, a calcium metal salt is affixed on the surface of a slide, multi-well plate, or other surface to form a transfection device. By using this device, people need only add nucleic acid to the surface and allow the calcium metal salt to form a complex with the nucleic acid. This reaction occurs in approximately 30 minutes, then cells are seeded on the surface and incubated under suitable conditions for introduction of the nucleic acid(s) into the cells.

Any suitable surface that can be used to affix the nucleic acid-containing mixture to its surface can be used. For example, the surface can be glass, plastics (such as polytetrafluoroethylene, polyvinylidenedifluoride, polystyrene, polycarbonate, polypropylene), silicon, metal, (such as gold), membranes (such as nitrocellulose, methylcellulose, PTFE or cellulose), paper, biomaterials (such as protein, gelatin, agar), tissues (such as skin, endothelial tissue, bone, cartilage), minerals (such as hydroxylapatite, graphite). According to preferred embodiments the surfaces may be slides (glass or poly-L-lysine coated slides) or wells of a multi-well plate.

For slides, such as a glass slide coated with poly-L-lysine (e.g., Sigma, Inc.), the calcium metal salt is fixed on the surface and dried, and then a nucleic acid of interest or a nucleic acid to be introduced into cells is introduced. The slide is incubated at room temperature for 30 minutes to form nucleic acid/calcium metal salt complexes on the surface of the transfection device. The nucleic acid/calcium metal salt complexes create a medium for use in high throughput microarrays, which can be used to study hundreds to thousands of nucleic acids at the same time. In an alternative embodiment, the calcium metal salt is affixed on the surface of the transfection device in discrete, defined regions to form a microarray of calcium metal salts. In this embodiment, nucleic acids, which are to be introduced into cells, are spread on the surface of the transfection device along with a calcium metal salt. This method can be used in screening calcium metal salts from thousands of compounds. The results of such a screening method can be examined through computer analysis.

In another embodiment of the invention one or more wells of a multi-well plate may be coated with a calcium metal salt. Plates commonly used in transfection and drug screening are 96-well and 384-well plates. The calcium metal salt can be evenly applied to the bottom of plate. Hundreds of nucleic acids are then added into the well(s) by, for instance, a multichannel pipette or automated machine. Results of transfection are then determined by using a microplate reader. This is a very convenient method of analyzing the transfected cells, because microplate readers are commonly used in most biomedical laboratories. The multi-well plate coated with calcium metal salts can be widely used in most laboratories to study gene regulation, gene function, molecular therapy, and signal transduction, as well as drug screening. Also, if different kinds of calcium metal salts are coated on the different wells of multi-well plates, the plates can be used to screen many calcium metal salts relatively efficiently. Recently, 1,536 and 3,456 well plates have been developed, which may also be used according to the methods described herein.

The transfection reagent or delivery reagent used in the method of the invention are calcium metal salts which can introduce nucleic acids into cells. Preferred embodiments use calcium chloride or calcium acetate.

According to an one embodiment, the calcium metal salt can be mixed with a matrix, such as proteins, peptides, polysaccharides, or other polymers. The protein can be gelatin, collagen, bovine serum albumin or any other protein that can be used in affixing proteins to a surface. The polymers can be hydrogels, copolymers, non-degradable or biodegradable polymers and biocompatible materials. The polysaccharide can be any compound that can form a membrane and coat the calcium metal salt, such as chitosan. Other reagents, such as cytotoxicity reductive reagents, cell binding reagents, cell growing reagents, cell stimulating reagents or cell inhibiting reagents and the compounds for culturing specific cells, can be also affixed to the transfection device along with the calcium metal salt.

According to another embodiment, a gelatin-transfection reagent mixture, comprising a calcium metal salt such as calcium chloride and gelatin that is present in an appropriate solvent, such as water or double deionized water, may be affixed to the transfection device. In a further embodiment a cell culture reagent may also be present in the gelatin-transfection reagent mixture. The mixture is evenly spread onto a surface, such as a slide and multi-well plate, thus producing a transfection surface bearing the gelatin-transfection reagent mixture. In alternative embodiments, different transfection reagent-gelatin mixtures may also be spotted on discrete regions on the surface of the transfection device. The resulting product is allowed to dry completely under suitable conditions such that the gelatin-transfection reagent mixture is affixed at the site of application of the mixture. For example, the resulting product can be dried at specific temperatures or humidity or in a vacuum-dessicator.

The concentration of calcium metal salt present in the mixture depends on the transfection efficiency and cytotoxicity of the calcium metal salt. Typically there is a balance between transfection efficiency and cytotoxicity. At concentrations in which a calcium metal salt is most efficient, while keeping cytotoxicity at an acceptable level, the concentration of calcium metal salt is at the optimal level. The concentration of calcium metal salt will generally be in the range of about 1 to 10 mM, preferably about 2-7 mM. Similarly, the concentration of gelatin or other matrix depends on the experiment or assay to be performed, but the concentration will generally be in the range of 0.1% to 5%, preferably 0.5 to 2% and most preferably about 1-2% of the calcium metal salt. According to embodiments shown in the examples, the gelatin concentration is about 2% of the calcium metal salt. Of course, the concentration of the calcium metal salt in the gel matrix will be higher than in the total reaction volume - on the order of 5mM to 0.1 M, preferably 10-40 mM. In a preferred embodiment, the calcium metal salt in the matrix is applied to the transfection device and allowed to dry in a clean atmosphere. Preferably, the calcium metal salt in the matrix is dried for two hours to overnight in a sterile hood.

The molecules to be introduced into cells are nucleic acids, including peptide nucleic acid (PNA). The nucleic acid can be DNA, RNA and DNA/hybrid, etc. If the DNA used is present in a vector, the vector can be of any type, such as a plasmid (e.g. example, pCMV-GFP, pCMV-luc) or viral-based vector (e.g. pLXSN). The DNA can also be linear fragment with a promoter sequence (such CMV promoter) at the 5' end of the cDNA to be expressed and a poly A site at the 3' end. These gene expression elements allow the cDNA of interest to be transiently expressed in mammalian cells. If the DNA is the single strand oligodeoxynucleotide (ODN), for example antisense ODN, it can be introduced into cells to regulate target gene expression. In embodiments using RNA the nucleic acid may be single stranded (antisense RNA and ribozyme) or double stranded (RNA interference, siRNA). In most cases, the RNA is modified in order to increase the stability of RNA and improve its function in down regulation of gene expression. In peptide nucleic acid (PN_{A}), the nucleic acid backbone is replaced by peptide, which makes the molecule more stable.

Under appropriate conditions, the nucleic acids are added into the transfection device, which has been coated with calcium metal salt(s), to form biomolecule/delivery reagent complexes. The nucleic acids are preferably dissolved in cell culture medium without fetal bovine serum and antibiotics, for example Dulbecco's Modified Eagles Medium (DMEM) or opti-MEM. If the calcium metal salt is evenly affixed on the slide, the nucleic acid can be spotted onto discrete locations on the slide. Alternatively, calcium metal salt may be spotted on discrete locations on the slide, and the nucleic acid can simply be added to cover the whole surface of the transfection device. If the calcium metal salt is affixed on the bottom of multi-well plates, the nucleic acids are simply added into different wells by multi-channel pipette, automated device, or other method. The resulting product (transfection device coated with calcium metal salt and nucleic acid) is incubated to form the bio-molecule/transfection reagent (or delivery reagent) complexes. In some embodiments, the incubation is for approximately 25 minutes room temperature. In some cases, for example, different kinds of nucleic acids are spotted at discrete locations on the slide, and the DNA solution will be removed after incubation to produce a surface bearing nucleic acids in complex with calcium metal salt. In other embodiments, the nucleic acid solution can be kept on the surface.

After adding the nucleic acid, cells in an appropriate medium and appropriate density are plated onto the surface. The resulting product (a surface bearing nucleic acid and plated cells) is maintained under conditions that result in entry of the nucleic acid into plated cells.

Suitable cells for use according to the methods described herein include yeast, or higher eukaryotic cells, including plant and animal cells, especially mammalian cells. Eukaryotic cells, such as mammalian cells (e.g., human, monkey, canine, feline, bovine, or murine cells), insect or plant cells, are plated onto the transfection device, which is coated with calcium metal salt and nucleic acid, in sufficient density and under appropriate conditions for introduction/entry of the nucleic acid into the eukaryotic cells and either expression of the DNA or interaction of the nucleic acid with cellular components. In particular embodiments, the cells may be selected from hematopoietic cells, neuronal cells, pancreatic cells, hepatic cells, chondrocytes, osteocytes, or myocytes. The cells can be fully differentiated cells or progenitor/stem cells.

in preferred embodiments, eukaryotic cells are grown in Dulbecco's Modified Eagles Medium (DMEM) containing 10% heat-inactivated fetal bovine serum (FBS) with L-glutamine and penicillin/streptomycin (pen/strep). It will be appreciated by those of skill in the art that certain cells should be cultured in a special medium, because some cells need special nutrition, such as growth factors and amino acids. The optimal density of cells depends on the cell types and the purpose of experiment. For example, a population of 70-80% confluent cells is preferred for gene transfection, but for oligonucleotide delivery the optimal condition is 30-50% confluent cells. In an example embodiment, if 5x10⁴ 293 cells/well were seeded onto a 96 well plate, the cells would reach 90% confluency at 18-24 hours after cell seeding. For HeLa 705 cells, only 1×10⁴ cells/well are needed to reach a similar confluent percentage in a 96 well plate.

After the cells are seeded on the surface containing nucleic acid/calcium metal salt, the cells are incubated under optimal conditions for the cell type (e.g. 37°C, 5-10% CO₂). The culture time is dependent on the purpose of experiment. Typically, the cells are incubated for 24 to 48 hours for cells to express the target gene in the case of gene transfection experiments. In the analysis of intracellular trafficking of biomolecules in cells, minutes to several hours of incubation may be required and the cells can be observed at defined time points.

The results of nucleic acid delivery can be analyzed by different methods. In the case of gene transfection and antisense nucleic acid delivery, the target gene expression level can be detected by reporter genes, such as green fluorescent protein (GFP) gene, luciferase gene, or β-galactosidase gene expression. The signal of GFP can be directly observed under a fluorescence microscope, the activity of luciferase can be detected by a luminometer, and the blue product catalyzed by β-galactosidase can be observed under microscope or determined by a microplate reader. One of skill in the art is familiar with how these reporters function and how they may be introduced into a gene delivery system. The nucleic acid and its product, the protein, peptide, and the target modulated by these biomolecules can be determined by various methods, such as detecting immunofluorescence or enzyme immunocytochemistry, autoradiography, or in situ hybridization. If immunofluorescence is used to detect expression of an encoded protein, a fluorescently labeled antibody that binds the target protein is used (e.g., added to the slide under conditions suitable for binding of the antibody to the protein). Cells containing the protein are then identified by detecting a fluorescent signal. If the delivered molecules can modulate gene expression, the target gene expression level can also be determined by methods such as autoradiography, in situ hybridization, and in situ PCR. However, the identification method depends on the properties of the delivered nucleic acids, their expression product, the target modulated by it, and/or the final product resulting from delivery of the nucleic acids.

### EXAMPLES

### Example 1

### Preparation of Cell Culture/Transfection Device

Calcium chloride and linear polyethylene imine (L-PEI) were used for transfecting plasmid DNA into mammalian cells in vitro to evaluate the transfection efficiency. The transfection reagents were affixed on the well bottoms of a 96-well cell culture plate (Corning Costar, Cat. No. 3997) with gelatin (Type B; SIGMA-ALDRICH, Cat. No. G-9391). In the case of calcium chloride, 0.5 µmol/well of calcium chloride was affixed with 0.₅ mg/well of gelatin. In the case of PEI, 3.5 µg/well of PEI was affixed with 0.05 mg/well of gelatin. These reagents and gelatin were dissolved in 25 µl of deionized water (per each well) and distributed to the wells. The wells were then air-dried.

### Example 2

### Transfection with Cell Culture/Transfection Device for 293 Cells

50 ng of pCMV-GFP plasmid in 25 µl of opti-MEM I (Invitrogen, Cat. No. 31985-070) was added in each well and kept at room temperature for 25 minutes. Next, 5 X 10⁴ of 293 cells in 100 µl of Dulbecco's modified Eagle Medium (DMEM) (Invitrogen, Cat. No. 11965-084) with 10% calf serum (Invitrogen) were added and incubated at 37°C in 7.5% CO₂. After 24 h incubation, transfection efficiency was estimated by observing GFP fluorescence by using an epifluorescent microscope (IX70, Olympus). Transfection efficiencies are shown in Table 1. Both transfection reagents showed very high transfection efficiency.

**Table 1**

| Transfection Efficiency with Transfectable Cell Culture Device | |
|---|---|
| Transfection Reagent | GFP-positive cells |
| L-PEI | 60% |
| CaCl₂ | 70% |
| | |

### Example 3

### Conventional Transfection for 293 Cells

100 µl of 293 cell culture (5 X 10⁵ cells/ml in DMEM) was seeded in a 96-well plate and incubated at 37 °C in 7.5% CO₂ for 24h. After the cultivation, pCMV-GFP plasmid-transfection reagent mixture (final concentration: plasmid; 10 ng/µl, transfection reagent; as written in Table 2), dissolved in opti-MEM, was prepared and incubated at room temperature for 15 min to allow forming plasmid-transfection reagent complex. Then, 15 µl of plasmid-transfection reagent complex was added in each well and incubated at 37 °C in 7.5% CO₂ for 24h. Transfection efficiency was estimated as described in Example 2. As a result, L-PEI was effective in conventional method; however, calcium chloride was not suitable. In the conventional method, two incubation steps and a complicated preparation are required.

As can be seen from Table 2, CaCl₂ was not an effective transfection agent when used in a conventional method. L-PEI was an effective agent although transfection efficiencies were lower than observed in the experiment of Example 2 (Table 1).

**Table 2**

| Transfection Efficiency in Conventional Protocol | | |
|---|---|---|
| Transfection Reagent | Amount (per each well) | GFP-positive "Cells |
| L-PEI | 4.8 µg | 35% |
| | 2.4 µg | 40% |
| | 1.2 µg | 40% |
| | 0.60 µg | 30% |
| CaCl₂ | 0.38 µmol | 1% |
| | 0.28 µmol | 0% |
| | 0.19 µmol | 0% |
| | 0.094 µmol | 0% |

### Example 4

### Multiple Cell Lines Assay

Transfection efficiencies on mammalian cells (293 cells, COS-7 cells and HeLa cells) were measured. A cell culture/transfection device was prepared by affixing calcium chloride and gelatin with various conditions on the bottom of a 96-well plate. Transfection of pCMV-GFP was carried out by following the protocol written in Example 2. However, number of seeded cells was 2 X 10⁴ in 100 µl of DMEM with 10% calf serum for COS-7 and HeLa cells, and 5 X 10⁴ in 100 µl of DMEM with 10% calf serum for 293 cells.

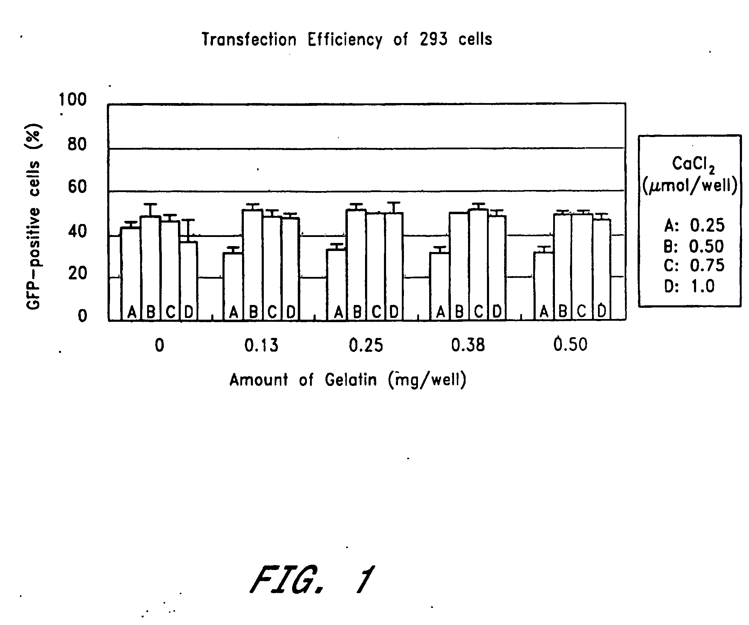

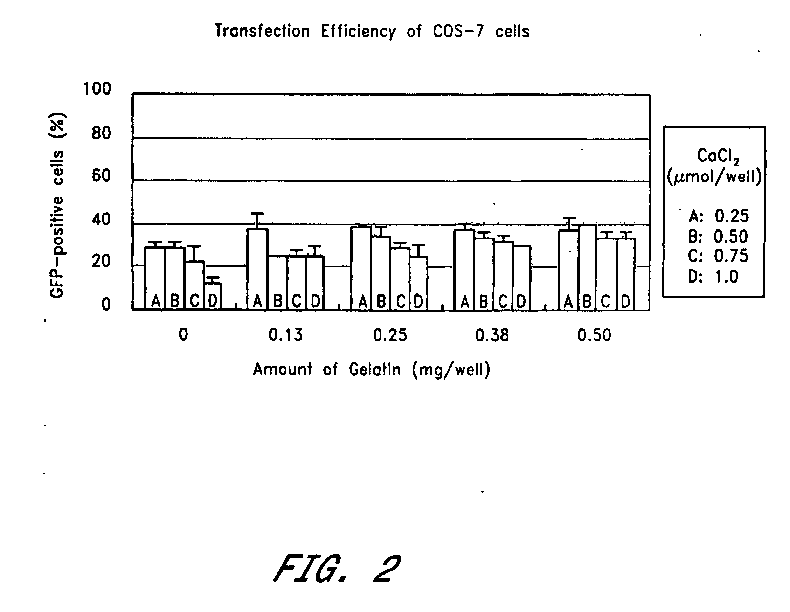

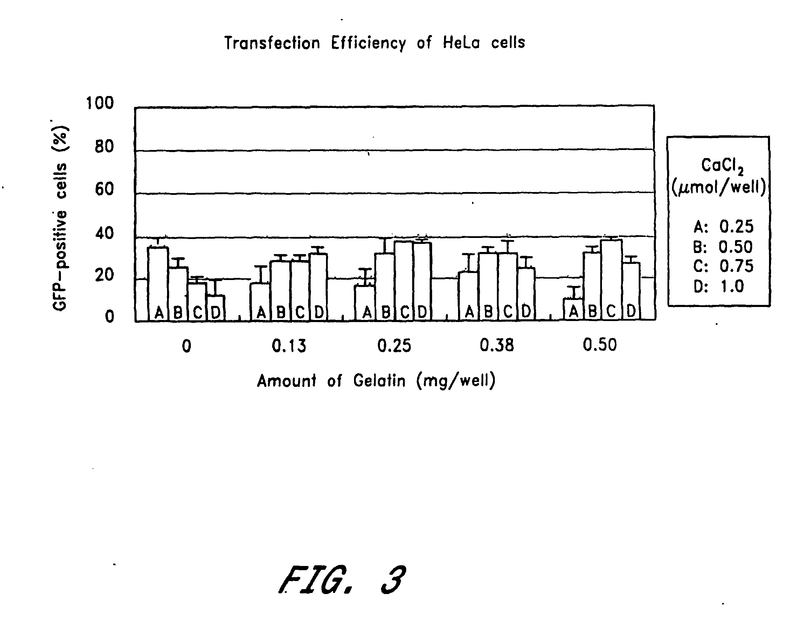

Figures 1, 2 and 3 show the transfection efficiency by using the 96-well transfection plate. pCMV-GFP plasmid was transfected into every cell line by using this pretreated plate, and this result shows the applicability of the cell culture/transfection plate for a range of commonly cultured mammalian cell types.

### Example 5

### siRNA Delivery by Using Cell Culture/Transfection Device

siRNA is an important tool in the life science research area to investigate the role of specific genes. The methodology described above for the cell culture/transfection device was applied to siRNA delivery.

GT2-293-LUC cell, which has luciferase gene GL2, was purchased from BD Biosciences Clonetech, Palo Alto, CA USA and siRNA was purchased from Dharmacon Inc., Lafayette, CO USA. Sequence of siRNA which targeted GL2 is as follows:
Sense sequence: CGUACGCGGAAUACUUCGAdTdT (SEQ ID NO: 1)
Antisense sequence: UCGAAGUAUUCCGCGUACGdTdT (SEQ ID NO: 2)
These two fragments were annealed to form a double-strand.

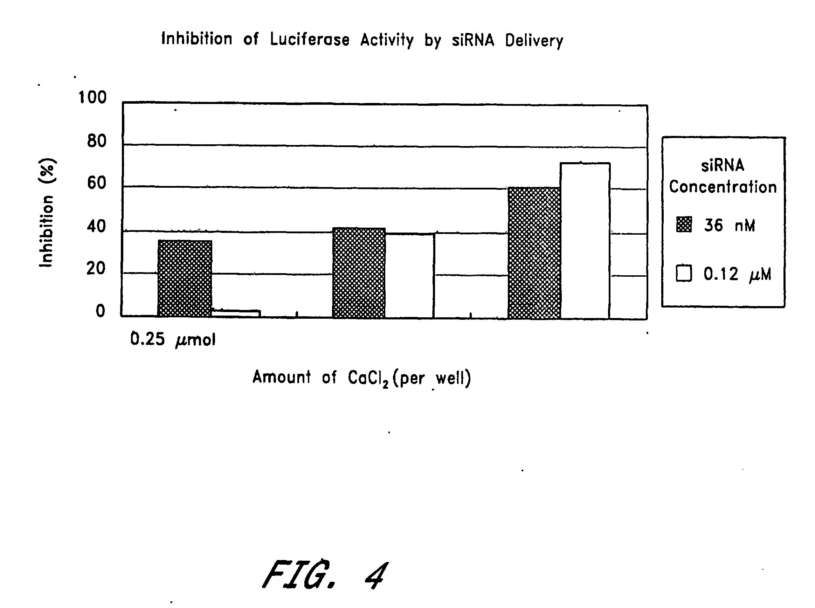

The cell culture/transfection device was prepared as follows: 25 µl of 2% gelatin type B plus 10, 20 or 30 mM CaCl₂ solution was put in a 96-well plate and dried overnight. Therefore, the bottom of each well was covered with 0.5mg of gelatin and 0.25, 0.50 or 0.75 µmol of CaCl₂. Then, 25 µl of siRNA solution (36 nM or 0.12 µM in opti-MEM) were added in each well and incubated for 25 min. 100 µl of GP2-293-LUC cells (5 X 10⁵ cells/ml in DMEM with 10% calf serum) were added and incubated at 37 °C in 7.5% CO₂ for 48h. Luciferase activity of cells were determined by using a Dynex MLX Microtiter® plate luminometer and Luciferase Assay System (Promega Corp. Madison, Wl USA). The effect of siRNA was evaluated by comparing luciferase activity of transfected cells with untreated cells (Figure 4). The siRNA interfered with luciferase activity of GP2-293-LUC cells by up to 70%. Thus, the invention is also applicable to siRNA delivery.

### Example 6

### Co-transfection by Using Cell Culture/Transfection Device

In typical viral vector production, 2 or more plasmids are transfected into cells. Co-transfection can simulate the efficacy of cell culture/transfeclon device for viral vector production.

pCMV-luc plasmid, which carries luciferase gene, and pCMV-GFP were co-transfected into mammalian cells. The cell culture/transfection device was prepared as follows: 25 µl of 2% gelatin type B plus 20mM CaCl₂ solution was put into wells of a 96-well plate and dried overnight. Therefore, the bottom of each well was covered with 0.5mg of gelatin and 0.50 µmol of CaCl₂. Then, 25 µl of plasmid solution (in opti-MEM) was added in each well and incubated for 25 min. The amount of plasmids per 25 µl of solution was as follows: (1) 500 ng of pCMV-GFP and 500 ng of pCMV-luc; (2) 250 ng of pCMV-GFP and 250 ng of pCMV-luc; (3) 500 ng of pCMV-GFP; (4) 250 ng of pCMV-GFP; (5) 500 ng of pCMV-luc; and (6) 250 ng of pCMV-luc. Then, 100 µl of cell culture (293 cells: 5 X 10⁵ cells/ml, COS-7 cells: 2 X 10⁵ cells/mi, HeLa cells: 2 X 10⁵ cells/ml in DMEM with 10% calf serum) was added. After 24h incubation, transfection efficiency was estimated by observing GFP fluorescence and by measuring luciferase activity.

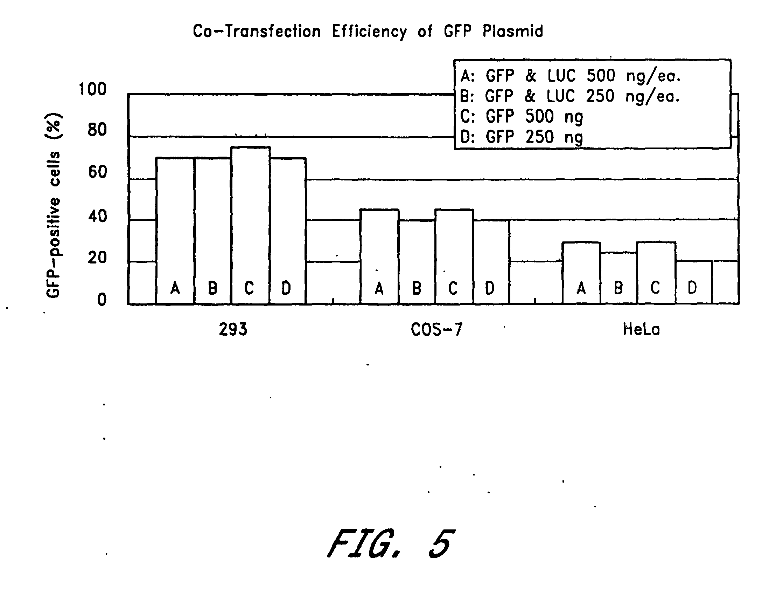

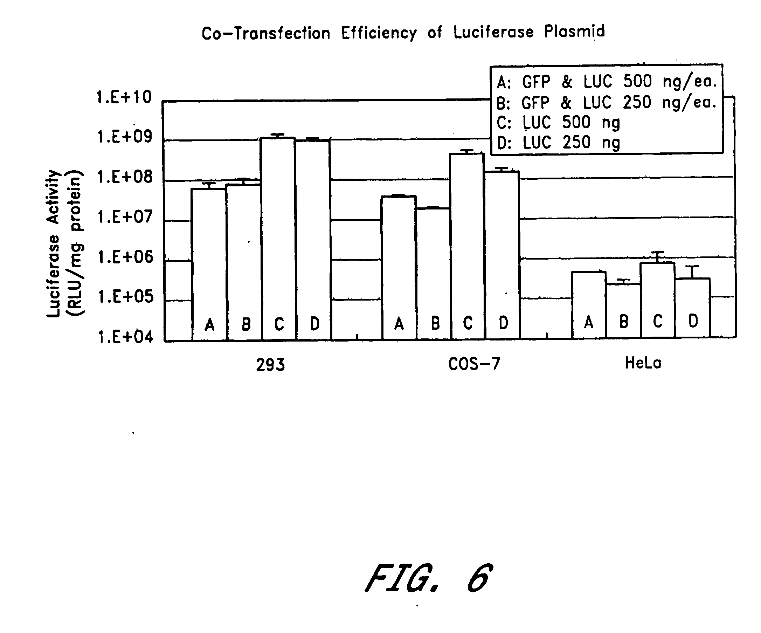

Figures 5 and 6 show the transfection efficiencies. Regarding GFP fluorescence, there were no obvious differences between normal (one plasmid) transfection and co-transfection in all cell lines. Luciferase activities of co-transfected 293 cells and COS-7 cells were 10-times lower than normally transfected cells, however, the activities of both co-transfected cells were still very high. These results indicate that the cell culture/transfection device described is applicable to co-transfection. Moreover this device is quite promising to viral vector production.

### Example 7

### Cell Culture/Transfection Device: Large Scale Applications

The cell culture/transfection device was also prepared for large scale applications. 24-well and 6-well plates are in common use and viral vector production is very important, especially for in vivo studies. For this purpose, transfection may be carried out in large cell culture dishes, for example 10cm or 25cm diameter.

The devices were prepared as follows: coating solution, containing 2% gelatin type B and 20mM CaCl₂ in deionized water, was put in each well or dish. The amount of coating solution added was: 1) 25 µl in 96-well plate, 2) 100 µl in 24-well plate, 3) 500 µlin 6-well plate, and 4) 2 ml in µl in 10cm dish. These plates and dishes were dried in a sterile hood.

After drying, 20 µg/ml of pCMV-GFP, dissolved in opti-MEM, was added to the plates and dishes. The amount of solution was: 1) 25 µl in 96-well plate, 2) 100 µl in 24-well plate, 3) 500 µl in 6-well plate, and 4) 3 ml in µl in 10cm dish. Then, plates and dishes were kept at room temperature for 25 min. Next, 293 cell culture was added to the plates and dishes, and incubated at 37 °C in 7.5% CO₂ for 24h. Finally, transfection efficiency was estimated by observing GFP fluorescence.

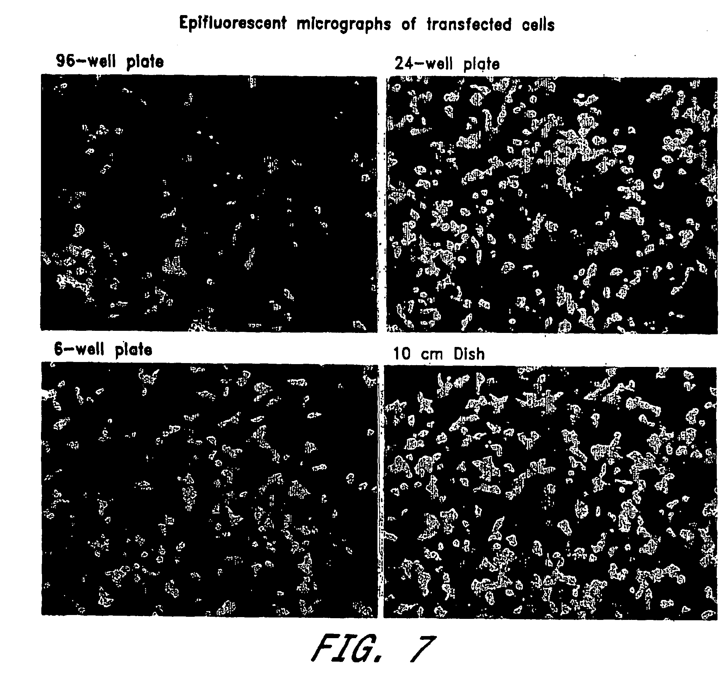

Figure 7 shows GFP fluorescence of transfected cells. Transfection efficiency was quite high in every plate or dish, and cell condition was also good. This data indicates that methods described herein are compatible with various sizes of cell culture devices.

## Claims

1. A method for transfection of eukaryotic cells comprising:
providing a solid surface at least partially coated with a calcium metal salt, optionally in a gel matrix;
adding at least one nucleic acid to be introduced into the eukaryotic cell onto the solid surface; and
seeding eukaryotic cells onto the solid surface at a sufficient density and under appropriate conditions for introduction of the nucleic acids into the eukaryotic cells.

2. The method of Claim 1, wherein the surface is selected from the group consisting of flasks, dishes, tubes, continuous surface, multi-well plates, slides, and implanted devices.

3. The method of Claim 1, wherein the solid surface is glass, polystyrene or epoxy resin.

4. The method of Claim 3, wherein the calcium metal salt is selected from the group consisting of calcium chloride and calcium acetate.

5. The method of Claim 1, wherein the calcium metal salt is in a gel matrix.

6. The method of Claim 5, wherein the calcium metal salt is at a concentration of 10-40 mM in a gel matrix.

7. The method of Claim 1, wherein the calcium metal salt is retained on the solid surface.

8. The method of Claim 7, wherein the calcium metal salt is retained on the solid surface with a matrix complex.

9. The method of Claim 8, wherein the matrix complex is selected from the group consisting of proteins, glycoproteins, peptides, polysaccharides, and polymers or combinations thereof.

10. The method of Claim 9, wherein said protein is selected from the group consisting of gelatin, collagen, laminin, fibronectin, and bovine serum albumin or a combination thereof.

11. The method of Claim 9, wherein said polymer is selected from the group consisting of hydrogels, biodegradable polymers, and biocompatible materials.

12. The method of Claim 1, wherein the solid surface is selected from the group consisting of a slide and a multi-well plate.

13. The method of Claim 1, wherein the at least one nucleic acid is selected from the group consisting of DNA, RNA, DNA/RNA hybrid and chemically modified nucleic acids.

14. The method of Claim 13, wherein the chemically modified nucleic acid comprises a peptide nucleic acid.

15. The method of Claim 13, wherein the DNA is circular, linear, or single strand oligonucleotide.

16. The method of Claim 13, wherein the RNA is single stranded or double stranded.

17. The method of Claim 16, wherein the single-stranded RNA is a ribozyme.

18. The method of Claim 16, wherein the double-stranded RNA is siRNA.

19. The method of Claim 1, wherein the cells are mammalian cells.

20. The method of Claim 1, wherein the cells are dividing cells or non-dividing cells.

21. The method of Claim 1, wherein the cells are transformed cells or primary cells.

22. The method of Claim 1, wherein the cells are somatic or stem cells.

23. The method of Claim 1, wherein the cell is a plant cell.

24. The method of Claim 1, wherein the cell is an insect cell.

## Patentansprüche

1. Verfahren für die Transfektion eukaryotischer Zellen, das umfasst:
Bereitstellen einer festen Oberfläche, die wenigstens partiell mit einem Calciummetallsalz beschichtet ist, gegebenenfalls in einer Gelmatrix,
Geben wenigstens einer Nucleinsäure, die in die eukaryotische Zelle eingebracht werden soll, auf die feste Oberfläche, und
Aussäen eukaryotischer Zellen auf die feste Oberfläche in ausreichender Dichte und unter Bedingungen, die für die Einbringung der Nucleinsäuren in die eukaryotischen Zellen geeignet sind.

2. Verfahren nach Anspruch 1, wobei die Oberfläche aus der Gruppe ausgewählt ist, die aus Flaschen, Platten, Röhren, kontinuierlichen Oberflächen, Multiwellplatten, Objektträgern und implantierten Vorrichtungen besteht.

3. Verfahren nach Anspruch 1, wobei die feste Oberfläche Glas, Polystyrol oder Epoxidharz ist.

4. Verfahren nach Anspruch 3, wobei das Calciummetallsalz aus der Gruppe ausgewählt ist, die aus Calciumchlorid und Calciumacetat besteht.

5. Verfahren nach Anspruch 1, wobei das Calciummetallsalz in einer Gelmatrix vorliegt.

6. Verfahren nach Anspruch 5, wobei das Calciummetallsalz in einer Konzentration von 10-40 mM in einer Gelmatrix vorliegt.

7. Verfahren nach Anspruch 1, wobei das Calciummetallsalz auf der festen Oberfläche zurückgehalten wird.

8. Verfahren nach Anspruch 7, wobei das Calciummetallsalz auf der festen Oberfläche mit einem Matrixkomplex zurückgehalten wird.

9. Verfahren nach Anspruch 8, wobei der Matrixkomplex aus der Gruppe ausgewählt ist, die aus Proteinen, Glycoproteinen, Peptiden, Polysacchariden und Polymeren oder Kombinationen davon besteht.

10. Verfahren nach Anspruch 9, wobei das besagte Protein aus der Gruppe ausgewählt ist, die aus Gelatine, Collagen, Laminin, Fibronectin und Rinderserumalbumin oder einer Kombination davon besteht.

11. Verfahren nach Anspruch 9, wobei das besagte Polymer aus der Gruppe ausgewählt ist, die aus Hydrogelen, biologisch abbaubaren Polymeren und biokompatiblen Materialien besteht.

12. Verfahren nach Anspruch 1, wobei die feste Oberfläche aus der Gruppe ausgewählt ist, die aus einem Objektträger und einer Multiwellplatte besteht.

13. Verfahren nach Anspruch 1, wobei die wenigstens eine Nucleinsäure aus der Gruppe ausgewählt ist, die aus DNA, RNA, DNA/RNA-Hybriden und chemisch modifizierten Nucleinsäuren besteht.

14. Verfahren nach Anspruch 13, wobei die chemisch modifizierte Nucleinsäure eine Peptidnucleinsäure umfasst.

15. Verfahren nach Anspruch 13, wobei die DNA ein ringförmiges, lineares oder einzelsträngiges Oligonucleotid ist.

16. Verfahren nach Anspruch 13, wobei die RNA einzelsträngig oder doppelsträngig ist.

17. Verfahren nach Anspruch 16, wobei die einzelsträngige RNA ein Ribozym ist.

18. Verfahren nach Anspruch 16, wobei die doppelsträngige RNA siRNA ist.

19. Verfahren nach Anspruch 1, wobei die Zellen Säugerzellen sind.

20. Verfahren nach Anspruch 1, wobei die Zellen sich teilende Zellen oder sich nicht teilende Zellen sind.

21. Verfahren nach Anspruch 1, wobei die Zellen transformierte Zellen oder primäre Zellen sind.

22. Verfahren nach Anspruch 1, wobei die Zellen somatische Zellen oder Stammzellen sind.

23. Verfahren nach Anspruch 1, wobei die Zelle eine Pflanzenzelle ist.

24. Verfahren nach Anspruch 1, wobei die Zelle eine Insektenzelle ist.

## Revendications

1. Procédé pour la transfection de cellules eucaryotes, comprenant les étapes consistant à :
fournir une surface solide au moins partiellement revêtue avec un sel métallique de calcium, éventuellement dans une matrice de type gel ;
ajouter au moins un acide nucléique devant être introduit dans la cellule eucaryote, sur la surface solide ; et
ensemencer des cellules eucaryotes sur la surface solide à une densité suffisante et dans des conditions appropriées pour l'introduction des acides nucléiques dans les cellules eucaryotes.

2. Procédé selon la revendication 1, dans lequel la surface est choisie dans le groupe constitué par les flacons, les plats, les tubes, une surface continue, les plaques multipuits, les lames et les dispositifs implantés.

3. Procédé selon la revendication 1, dans lequel la surface solide est un verre, un polystyrène ou une résine époxy.

4. Procédé selon la revendication 3, dans lequel le sel métallique de calcium est choisi dans le groupe constitué par le chlorure de calcium et l'acétate de calcium.

5. Procédé selon la revendication 1, dans lequel le sel métallique de calcium est dans une matrice de type gel.

6. Procédé selon la revendication 5, dans lequel le sel métallique de calcium est à une concentration de 10 à 40 mM dans une matrice de type gel.

7. Procédé selon la revendication 1, dans lequel le sel métallique de calcium est retenu sur la surface solide.

8. Procédé selon la revendication 7, dans lequel le sel métallique de calcium est retenu sur la surface solide avec un complexe de matrice.

9. Procédé selon la revendication 8, dans lequel le complexe de matrice est choisi dans le groupe constitué par les protéines, les glycoprotéines, les peptides, les polysaccharides, et les polymères ou combinaisons de ceux-ci.

10. Procédé selon la revendication 9, dans lequel ladite protéine est choisie dans le groupe constitué par la gélatine, le collagène, la laminine, la fibronectine et la sérumalbumine bovine ou une combinaison de ceux-ci.

11. Procédé selon la revendication 9, dans lequel ledit polymère est choisi dans le groupe constitué par les hydrogels, les polymères biodégradables et les matières biocompatibles.

12. Procédé selon la revendication 1, dans lequel la surface solide est choisie dans le groupe constitué par une lame et une plaque multipuits.

13. Procédé selon la revendication 1, dans lequel ledit au moins un acide nucléique est choisi dans le groupe constitué par un ADN, un ARN, un hybride ADN/ARN et les acides nucléiques modifiés chimiquement.

14. Procédé selon la revendication 13, dans lequel l'acide nucléique modifié chimiquement comprend un acide nucléique peptidique.

15. Procédé selon la revendication 13, dans lequel l'ADN est un oligonucléotide circulaire, linéaire ou simple brin.

16. Procédé selon la revendication 13, dans lequel l'ARN est simple brin ou double brin.

17. Procédé selon la revendication 16, dans lequel l'ARN simple brin est un ribozyme.

18. Procédé selon la revendication 16, dans lequel l'ARN double brin est un ARNi (pour « siRNA »).

19. Procédé selon la revendication 1, dans lequel les cellules sont des cellules de mammifère.

20. Procédé selon la revendication 1, dans lequel les cellules sont des cellules se divisant ou des cellules ne se divisant pas.

21. Procédé selon la revendication 1, dans lequel les cellules sont des cellules transformées ou des cellules primaires.

22. Procédé selon la revendication 1, dans lequel les cellules sont des cellules somatiques ou souches.

23. Procédé selon la revendication 1, dans lequel la cellule est une cellule de plante.

24. Procédé selon la revendication 1, dans lequel la cellule est une cellule d'insecte.


### SEQUENCE LISTING

<110> Nitto Denko Corporation
   Tanaka, Yasunobu
   Yu, Lei
   Ji, Shouping
<120> CULTURE DEVICE AND METHOD FOR EUKARYOTIC CELL TRANSFECTION
<130> NDTCO.029VPC
<160> 2
<170> FastSEQ for Windows Version 4.0
<210> 1
   <211> 21
   <212> RNA
   <213> Artificial Sequence
<220>
   <223> sense sequence of siRNA to target GL2
<221> misc RNA
   <222> (1) ... (21)
   <223> first 19 residues are RNA; last 2 residues are DNA
<400> 1
   cguacgcgga auacuucgat t 21
<210> 2
   <211> 21
   <212> RNA
   <213> Artificial Sequence
<220>
   <223> antisense sequence of siRNA to target GL2
<221> misc_signal
   <222> (1)...(21)
   <223> first 19 residues are RNA; last 2 residues are DNA
<400> 2
   ucgaaguauu ccgcguacgt t 21